Europäisches Patentamt

**European Patent Office**

**Office européen des brevets**

(11) Veröffentlichungsnummer: **0 003 544**
**B1**

(12) **EUROPÄISCHE PATENTSCHRIFT**

(51) Int. Cl.³: **A 61 K 7/28,** A 23 L 1/34,
A 23 G 3/00, A 23 K 1/165

(45) Veröffentlichungstag der Patentschrift:
**04.11.81**

(21) Anmeldenummer: **79100248.8**

(22) Anmeldetag: **29.01.79**

(54) **Karieshemmende, wasserarme, zuckerhaltige, speziell saccharosehaltige Lebensmittel und karieshemmende, wasserarme, zuckerfreie pharmazeutische Zubereitungen, sowie Verfahren zu deren Herstellung.**

(30) Priorität: **31.01.78 DE 2804138**

(43) Veröffentlichungstag der Anmeldung:
**22.08.79 Patentblatt 79/17**

(45) Bekanntmachung des Hinweises auf die Patenterteilung:
**04.11.81 Patentblatt 81/44**

(84) Benannte Vertragsstaaten:
**BE FR IT LU**

(56) Entgegenhaltungen:
**FR-A-2 104 349
GB-A-1 272 135
CHEMICAL ABSTRACTS, Vol 69
1968, Columbus, Ohio, USA,
DRAUS et al.: »Salivary
enzymes and calculus formation«,
Seite 8714, rechte Spalte**

(73) Patentinhaber: **Ferrero oHG, D-3570 Stadtallendorf (DE)**

(72) Erfinder: **Lembke, Andreas, Prof. Dr. Dr., Institut für
Virusforschung u. experiment. Medizin,
D-2420 Eutin-Sielbeck (DE)**
Erfinder: **Gorny, Dietrich, Weiner Strasse 75,
D-6000 Frankfurt/Main (DE)**

(74) Vertreter: **Eitle, Werner, Dipl.-Ing. et al, Arabellastrasse 4,
D-8000 München 81 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Karieshemmende, wasserarme, zuckerhaltige, speziell saccharosehaltige Lebensmittel und karieshemmende, wasserarme, zuckerfreie pharmazeutische Zubereitungen, sowie Verfahren zu deren Herstellung

Die Erfindung betrifft karieshemmende, wasserarme, zuckerhaltige, speziell saccharosehaltige Lebensmittel und karieshemmende, wasserarme, zuckerfreie pharmazeutische Zubereitungen, insbesondere Zahn- und Mundpflegemittel, sowie Verfahren zu deren Herstellung.

Es ist bekannt, daß es im Mundraum in Gegenwart von Mikroorganismen durch Nahrungs-, Genuß- und Arzneimittel mit einem hohen Gehalt an Rohrzucker zur Ausbildung sogenannter »Plaques« kommt und saure Abbauprodukte entstehen, die den Zahnschmelz stark angreifen. Diese Einwirkung ist unter dem Namen Karies bekannt. Man versteht darunter einen zunächst oberflächlichen Befall der Zähne, welche dann fortschreitend unter Bildung von Kavitäten zerstört werden.

Für die Entstehung der Zahnkaries sind in erster Linie diejenigen Mikroorganismen von Bedeutung, welche sich in bzw. unter den als Plaques bezeichneten Zahnbelägen finden. Die in bzw. unter den Zahnplaques vorhandenen Mikroorganismen leben, wie alle anderen in der Mundhöhle vorhandenen Keime, von den dort nach der Nahrungsaufnahme verbleibenden Speiseresten. Besonders willkommen sind den Bakterien niedermolekulare Kohlehydrate, vor allem verschiedene Zuckerarten, weil diese die besten Energiequellen für ihre intensiven Wachstums- und Stoffwechselvorgänge darstellen.

Der Mechanismus der Kariesbildung scheint noch nicht in allen Einzelheiten exakt geklärt. Die kariogenen Mikroben, z. B. Streptococcus mutans, bilden das Ferment Dextransaccharase aus, welches den Rohrzucker( = Saccharose) in Anhydroglucose und Fructose zerlegt. Die Anhydroglucose kann sich dann zu polymeren Molekülen zusammenlagern, die als Dextrangele bezeichnet werden. Unter diesen weisen insbesondere Dextrangele mit einem Molekulargewicht von 10 000 bis 200 000 eine besondere Klebkraft auf. Diese Dextrangele dienen gewissermaßen als »Klebemittel«, die die betreffenden Bakterien auf den Zahnoberflächen festhalten, d. h. die Plaques ausbilden. Die genannten Bakterien können dann unter den Plaques unter Luftabschluß, z. B. aus vorhandenem oder eindiffundiertem Zucker, Säure, wie z. B. Milchsäure oder andere zahnzerstörende Säuren erzeugen, was zur Entstehung erster Schmelzläsionen (Initialkaries) führt, d. h. es kommt zu einer Korrosion des Dentins mit den bekannten Folgeerscheinungen. Nach Ausbildung der Plaques wird bei der hierunter erfolgenden kariösen Demineralisierung, insbesondere aus dem Schmelzapatit des Zahns, unter Einwirkung von Säure allmählich Kalzium und Phosphat herausgelöst.

Bei den bisherigen Versuchen zur Hemmung von Karies sind zwei Wege eingeschlagen worden. Diese bestehen grundsätzlich entweder in der Erhöhung des Widerstandes der Zahnhartsubstanzen gegenüber dem Säureangriff oder in der Abschwächung des Säureangriffs auf den Zahn.

Unter die erstgenannte Lösung können Versuche der Trinkwasser-, Salz- oder Milchfluoridierung zusammengefaßt werden, die in breitem Umfange vor allem in den USA und der Schweiz durchgeführt worden sind. Daneben sind auch die Lokalapplikation von fluoridhaltigen Tabletten bzw. die Anwendung fluorierter Verbindungen, wie Natriumfluorid, Natriummonofluorphosphat, Zinnfluorid-Kalziumpyrosphat oder Aminfluorid erwähnenswert. Wenngleich nach Berichten hierdurch eine Abnahme kariöser Läsionen berichtet worden ist, so können die erzielten Ergebnisse jedoch nicht befriedigen. Daneben bestehen gegen die Chemikalieneinbringung insbesondere in Trinkwasser und Nahrungsmittel, wie Milch, erhebliche Vorbehalte auch physiologischer Art. Auch reichen kollektive und individuelle Fluoridierungsmaßnahmen bei der derzeitigen Ernährung der Zivilbevölkerung nicht aus, um dem kariösen Verfall der Zähne entgegenzuwirken.

Eine Kariesbekämpfung ist aber auch durch Kontrolle der Dextranbildung, beispielsweise durch Einwirkung von Chemikalien, versucht worden. So ist beispielsweise in »Journal of Dental Health«, Band 22, Nr. 4, Dezember 1972, die Verwendung von Natriumoleat und -linoleat zu diesem Zweck beschrieben worden, deren Wirkung auf die Bildung von Dextran gemäß der DE-OS-2 442 825 durch wasserlösliche, sekundäre Phosphate in Mitteln zur Zahnhygiene noch gesteigert werden soll. Auch ist die Verwendung von Ca-, Na- oder Mg-Salzen von Estern der Phosphorsäure mit Saccharose, Glucose oder Lactose zur Herstellung von im Mundraum kariostatisch wirksamen Zahnpflegeprodukten in der DE-AS 1 467 809 in Betracht gezogen worden. Diese Maßnahmen der Blockierung des Metabolismus der Mundmikroflora, die das Dextran-Saccharose-Enzym-System zur Erzeugung der »Dental-Plaques« verwenden, haben zwar eine gewisse Verbesserung der Zahnpflegemittel, nicht jedoch die gewünschte einschneidende Bekämpfung des kariösen Zahnverfalls mit sich gebracht.

Auch die bekannten Versuche der Ausnutzung des Lyolyse-Prinzips, nämlich der Karies entweder durch Zerstörung der kariogenen Mikroorganismen durch deren Auf- bzw. Anlösung oder durch die Auflösung bzw. Anlösung der klebenden Dextrangele vorzubeugen, konnten bislang nicht befriedigen. So ist in der DE-AS 2 011 935 die Zerstörung bzw. der Angriff kariogener Mikroben durch Enzyme beschrieben, die aus bestimmten Streptococcen-Stämmen isoliert worden sind. Zu diesem Zweck sind drei definierte Stämme und deren Einbringung in Zahnpflegemittel angegeben worden. Daneben ist auch das Lyolyseprinzip auf bereits gebildetes Dextran mittels des Enzyms Dextranase gemäß der DE-OS 1 955 956 angewandt worden. Nach der AT-PS 318 815 vermag die Dextranase allerdings nur lösliches Dextran zu zersetzen, da das unlösliche Dextran einen Mutan-Anteil aufweist, der durch Dextranase nicht angegriffen werden kann. Daher ist in dieser österreichischen Druckschrift der Einsatz von »Mutanase« vorgeschlagen worden.

Jedoch auch diese Versuche der Verbesserung von Zahnpflegemitteln durch Auflösung der Dextran erzeugenden Mikroorganismen oder der Dextrane selbst und der Inkorporierung derartiger Systeme in Zahnpflegemittel haben bislang noch nicht vollständig befriedigen können. Dies ist zum Teil darauf zurückzuführen, daß eine Pflege der Zähne nach jeder Mahlzeit, der Einnahme von Süßigkeiten etc. nicht immer möglich ist, um die zwischenzeitlich aufgetretenen Schädigungen hintenan zu halten.

Einen weiteren Weg zur Bekämpfung der Karies sah man in dem teilweisen bzw. vollständigen Ersatz von Rohrzucker durch andere, nicht-kariogene Zucker oder Zuckeralkohole bzw. durch synthetische Süßungsmittel.

Ein vollständiger Ersatz der Saccharose durch wenig bzw. nicht-kariogene Zuckeraustauschstoffe, z. B. durch die Zuckeralkohole Xylit und Sorbit, kommt aus technologischen, wirtschaftlichen, organoleptischen oder medizinischen Erwägungen bei vielen Anwendungen nicht in Frage.

Unter den synthetischen Süßstoffen besitzt Saccharin, einer der bekanntesten und am häufigsten verwendeten Zuckerersatzstoffe, den Nachteil, beim Verbraucher einen unangenehmen Nachgeschmack zu erzeugen. Bei anderen Stoffen, z. B. den Cyclamaten, macht die Ärzteschaft ernsthafte Vorbehalte hinsichtlich ihrer Schädlichkeit. Die synthetischen Süßstoffe haben andererseits den Nachteil, daß sie wegen der hohen Süßkraft einzusetzenden kleinen Mengen nicht leicht zu dosieren sind, z. B. spricht man in diesem Zusammenhang von einem »leeren Geschmack«, d. h. es fehlt der erwünschte »Körper«. Auch physiologische Wirkungen sind der allgemeinen Verbreitung dieser synthetischen Süßstoffe hinderlich.

Es wurde auch der Einsatz von Enzymen empfohlen, um den Abbau von Speiseresten zu beschleunigen und dadurch die Bildung von Plaques zu verhindern. Angegeben werden α-Glucosidasen und/oder β-Fructosidasen (DE-OS 1 927 411), sowie eine Polymer-Enzym-Verbindung, die neutrale alkalische und saure Proteasen neben Amylase, Lipase, Dextranase enthält (DE-OS 1 948 298).

Aus der DE-OS 2 027 019 sind Zahnpflegemittel bekannt, die wasserarm, zuckerhaltig und dadurch karieshemmend sind, daß sie Enzyme, wie Glucoseoxidase und Lactatoxidase enthalten. Entscheidend hierbei ist, daß diese Oxido-Reduktasen das Substrat mit Sauerstoff unter Bildung von Wasserstoffperoxid zersetzen. Durch dieses enzymatisch gebildete aggressive Agens werden die am kariogenen Angriff beteiligten Mikroorganismen oxidativ geschädigt bzw. zerstört. Hierdurch wird aber die Mundflora insgesamt durch an sich körperfremde Medien angegriffen, was nachteilig ist.

In Chemical Abstracts 69 (1968) 93235 t ist der Einfluß verschiedener Enzyme auf die Zahnsteinbildung behandelt. Unter diesen Enzymen ist auch das Enzym Lactatdehydrogenase (LDH) genannt. Es konnte jedoch keine signifikante Korrelation zwischen der Aktivität von Lactatdehydrogenase und der Zahnsteinbildung festgestellt werden. Auch hat der Mechanismus der Zahnsteinbildung mit dem Problem der Karies keine direkte Relation. Die Bildung von Zahnstein stellt keinen Angriff auf das Zahnbein dar, noch ist dieser mit den sogenannten »Plaques« zu vergleichen, die maßgeblich die Bedingungen für den kariogenen Angriff mit schaffen. Ein Zusammenhang der referierten Untersuchungen mit dem Problem der Karieshemmung besteht nicht.

In der FR-PS 2 104 349 ist ein Verfahren zum homogenen Vermischen einer biologischen Festsubstanz mit Zucker beschrieben. Als Festsubstanz wird eine Reihe von Enzymen genannt, darunter auch das Enzym Lactatdehydrogenase. Durch die homogene Verteilung der Enzyme in Zucker, was durch Lyophilisierung einer entsprechenden Lösung zusammen mit Elektrolyten und Puffersubstanzen erreicht wird, wird versucht, die Enzyme zu stabilisieren. Das Problem der Reduzierung von Karies ist in der genannten Druckschrift nicht angesprochen.

In der GB-PS 1 272 135 ist die Verwendung einer Vielzahl von Enzymen in Mundpflegemitteln, Tierfutterzusammensetzungen und Waschpulvern angegeben. Die Enzyme werden dabei durch Zugabe eines Vehikels und eines enzymstabilisierenden Agens stabilisiert. Die stabilisierenden Agentien leiten sich von α,β-ungesättigten Säuren ab. Als Enzyme werden z. B. auch Oxidoreduktasen beschrieben, wie z. B. Glukoseoxidase, Xanthinoxidase, Alkoholdehydroginase. Das Verfahren der vorliegenden Druckschrift dient somit nur dem anderen Zweck, nämlich der Stabilisierung von Enzymen, welche insbesondere Waschmitteln zugesetzt werden.

Trotz zahlreicher Versuche zur Bekämpfung der Karies, die auf den unterschiedlichsten Prinzipien aufbauen, besteht noch immer die dringende Notwendigkeit an zusätzlich bzw. neuartigen Maßnahmen zur Verhinderung der schädlichen Folgen aus dem Säureangriff in Gegenwart kariogener Mikroorganismen, um bei der herkömmlichen Ernährung eine Zurückdrängung der Karies zu ermöglichen.

Aufgabe der vorliegenden Erfindung ist es, den schädlichen Wirkungen der Karies sowohl durch Verbesserung herkömmlicher wasserarmer Lebensmittel, die unter Verwendung von Zucker, speziell Saccharose, hergestellt worden sind, wie auch gegebenenfalls ergänzend durch eine gleichlaufende Verbesserung von Zahnpflegemitteln sowie zuckerfreien, pharmazeutischen Zubereitungen entgegenzuwirken. Die Verbesserung der Lebensmittel soll insbesondere dem Umstand Rechnung tragen, daß der kariogene Angriff bereits in den Zeitintervallen erfolgt, die zwischen den üblichen Zeiträumen der Zahnreinigung liegen. Insbesondere ist es erfindungsgemäß beabsichtigt, Lebensmittel ohne bzw. ohne vollständigen Ersatz der hierfür üblicherweise verwendeten Zucker durch den Zusatz einer physiologisch unbedenklichen Substanz in »karieshemmende« Lebensmittel umzuwandeln.

Diese Aufgabe wird erfindungsgemäß dadurch gelöst, daß die vorangehend genannten, wasserarmen, zuckerhaltigen, speziell saccharosehaltigen Lebensmittel und wasserarmen, zuckerfreien pharmazeutischen Zubereitungen, insbesondere Zahn- und Mundpflegemittel, einen Gehalt an Lactatdehydrogenase aufweisen.

Überraschenderweise hat sich bei Versuchen gezeigt, daß durch Zusatz des Enzyms Lactatdehydrogenase der Kariesbefall stark abnimmt. Es wird angenommen, daß durch diesen Lactatdehydrogenasezusatz bei wasserarmen bzw. wasserfreien Lebensmitteln bzw. Süßwaren ein Hemmsystem eingebaut wird, das bei deren Verzehr dem schädlichen Einfluß der durch Zuckerabbau entstandenen Produkte in Gegenwart kariogener Bakterien entgegenwirkt.

»Wasserarme« Lebensmittel sind solche Lebensmittel, deren Feuchtigkeitsgehalt sehr gering ist. Vorzugsweise werden damit Lebensmittel mit einer Restfeuchte von 0,1 bis 2% verstanden.

Unter »zuckerhaltigen« Lebensmitteln sind solche Lebensmittel zu verstehen, die einen Anteil natürlich vorkommender Zucker, wie Saccharose, Glucose, Fructose etc., sowie als Zuckerersatzstoffe Zuckeralkohole enthalten. Bevorzugt sind insbesondere Lebensmittel mit einem Gehalt an Saccharose. Es ist bekannt, daß Saccharose gegenüber anderen Zuckerarten bei bestimmten wasserarmen Lebensmitteln erhebliche technologische Vorteile besitzt.

Gemäß der Erfindung können gegebenenfalls sogar auch solche Lebensmittel bzw. Süßwaren oder pharmazeutische Zubereitungen in Betracht gezogen werden, die keinen Gehalt an üblichen Zuckern aufweisen oder im wesentlichen zuckerfrei sind. Dies können Materialien sein, die auf der Grundlage von Zuckerersatzstoffen gesüßt sind, z. B. mit Saccharin, Cyclamat, aus tropischen Pflanzen extrahierten Proteinsüßstoffen, Xylit usw. Es kann sich jedoch auch um Produkte handeln, die im wesentlichen keinen Zuckeranteil von Natur aus aufweisen und auch einer maßgeblichen Süßung nicht bedürfen. Auch bei diesen Süßwaren ist die Einarbeitung von Lactatdehydrogenase vorteilhaft. Diese kann nämlich beim Verzehr des Lebensmittels mit den an den Zähnen haftenden Speiseresten anteilig im Mundraum verbleiben und dort z. B. aus Kohlehydratabbau resultierende kariogene Produkte unschädlich machen.

Der Ausdruck »wasserarme, zuckerhaltige Lebensmittel« soll jedoch insbesondere Süßwaren umfassen. Beispiele hierfür oder für nahestehende Produkte sind Backwaren, Desserts und Kunsthonig. Produkte von besonderem Interesse sind sogenannte Stimulanzien, wie verschiedene Arten von Süßigkeiten, d. h. Hart- und Weichkaramellen (Bonbons), Fondant, Schaumzuckerwaren, Gummizuckerwaren, Lakritzwaren, Dragees Fruchtpasten, Krokant, Brausepulver, Marzipan, Persipan, Nougat, Schokoladen- und Kakaoerzeugnisse, Lutscher, Pastillen, Kaugummi usw. Die Produkte vom Kaugummi-Typ sind von besonderem Interesse, da solche Produkte in Langzeitberührung mit dem Speichel stehen und eine lange Verweilzeit im Mund haben. Obwohl Kaugummis normalerweise nicht verschluckt werden, werden sie hier doch als Lebensmittel angesehen und fallen somit unter den Erfindungsgedanken.

Unter Langzeitberührung in Verbindung mit längeren Verweilzeiten versteht man für die oral zu verabreichenden Produkte Zeiten von wenigstens einigen Minuten.

Gemäß der Erfindung kann das Lebensmittel auch ein Tierfutter darstellen.

Nach einer anderen Ausführungsform der Erfindung wird Lactatdehydrogenase zuckerfreien pharmazeutischen Zubereitungen als Wirkstoff zugefügt, der natürlich auch ergänzend neben andere übliche Wirkstoffe treten kann. Als solche pharmazeutische Zubereitungen kommen vor allem Tabletten und Dragees in Frage, die pharmakologisch wirkende Substanzen enthalten. Unter Produkten, auf die die Erfindung anwendbar ist, können unterschiedliche Arten sogenannter quasi-medizinischer Produkte erwähnt werden, wie beispielsweise Hustenelixiere oder -sirup usw. Solche Produkte werden häufig vor dem Zubettgehen genommen, d. h. nachdem die Zähne bereits geputzt sind, und haben somit eine Langzeitwirkung auf den Zahnbelag.

Eine im Rahmen der Erfindung bevorzugte pharmazeutische Zubereitung stellen Mundhygienetabletten im weitesten Sinne dar, wie Zahnhygienedragees, Kautabletten bzw. zahnreinigende Kaugummis. Die vorteilhafte Wirkung des Wirkstoffes beruht auch hier darauf, daß sich aufgrund der Einspeichelung im Mundraum ein enzymatischer Schutzfilm über die Zähne legen kann, der die Umwandlung von etwa vorliegenden Zuckerresten zu kariogenen Produkten zu unterbinden vermag.

Die genannten Zahnpflegemittel können beispielsweise in Form einer Zahntablette vorliegen, die die üblichen Polier-, Binde-, Verdikkungs- und Feuchthaltemittel aufweist.

Geeignete Poliermittel, die beispielsweise für Zahnpflegemittel verwendbar sind, sind z. B. die üblichen Kalziumphosphate, wie Trikalziumphosphat, Alkalimetaphosphat, Magnesiumcarbonat, pulverförmige Kunststoffe, wie Polymethylmetacrylat, Harnstoff-Formaldehyd-Kondensationsprodukte etc. oder Gemische solcher Substanzen.

Daneben können die Zahnpflegemittel auch Konservierungsmittel, Aromastoffe und andere Hilfsstoffe enthalten. In Einzelfällen kann es jedoch auch erwünscht sein, durch den Zusatz von Lactatdehydrogenase die Wirkung anderer Aktivstoffe, die einen Kariesschutz aufgrund anderer Prinzipien bezwecken, zu unterstützen bzw. zu vervielfachen. Als solche üblichen Wirkstoffe können insbesondere Fluorverbindungen, wie Aminfluoride, Alkalifluoride etc. oder auch Dextranasen genannt werden.

Das erfindungsgemäß verwendete Enzym Lactatdehydrogenase (nachstehend auch als LDH bezeichnet) ist im Handel erhältlich. Lactatdehydrogenase kann unter anderem auch aus verschiedenen Mikroben, z. B. Hefe, hergestellt werden. Die aus Hefe gewonnene LDH ist verhältnismäßig stabil, z. B. bleibt eine in Glyzerin gelöste Enzympräparation bei +18°C über ein Jahr lang aktiv. Daneben kann LDH auch in Pufferlösungen in einem definierten pH-Bereich ohne nennenswerten Aktivitätsverlust über längere Zeit gelagert werden. Wie Versuche im Rahmen der Erfindung gezeigt haben, erleidet die in wasserarme bzw. wasserfreie Lebensmittel oder pharmazeutische Zubereitungen eingebrachte LDH während üblicher Lagerzeiten nur einen verhältnismäßig geringen Aktivitätsverlust, sofern ein solcher überhaupt auftritt.

Die Menge an Lactatdehydrogenase, die in das Lebensmittel, die Süßware oder die pharmazeutische Zubereitung wie Zahnreinigungstabletten, insbesondere jedoch in Pflegemittel für Zahnprothesen in Tablettenform, eingebracht wird, kann durch den Fachmann leicht aufgrund der spezifischen Enzymaktivität, des ungefähren Zuckeranteils des Materials, sofern ein solcher gegeben ist, sowie aufgrund des im Bindemittel bzw. bei dessen Herstellung vorliegenden pH-Bereiches ermittelt werden.

Erfindungsgemäß ist es bevorzugt, den Anteil an eingebrachter Enzymaktivität entsprechend der anzunehmenden Lagerzeit des Materials zumindest so zu bemessen, daß dieser im Moment des Verzehrs etwa ausreichend ist, um die gewünschte Wirkung hervorzurufen.

Im allgemeinen ist eine LDH-Menge von 0,2 mg bis 0,5 g pro kg geeignet. Vorzugsweise werden 0,5 mg bis 0,05 g pro kg verwendet, bezogen auf eine spezifische Aktivität von ca. 300 U/mg.

Außerdem sind hier noch die Temperatur und gegebenenfalls der Wassergehalt des Materials bei Zufügung des Enzyms bzw. dessen übliche Lagerungstemperatur in Betracht zu ziehen. Der zweckmäßigerweise einzubringende Anteil an Enzym kann durch einfache Versuchsansätze ermittelt werden.

Auch für pharmazeutische Zubereitungen und insbesondere Zahnpflege- bzw. Mundhygienemittel, in die Lactatdehydrogenase eingebracht werden kann, gilt, daß die einzubringende Enzymmenge durch überschlägige Berechnungen oder empirische Versuche ermittelt wird. In derartigen Zahnpflege- bzw. Mundhygienemitteln liegt üblicherweise kein Zuckeranteil vor, weshalb es nicht erforderlich ist, die einzubringende Enzymaktivität hieran zu orientieren. In diesem Fall dient die Einbringung von Enzym dem Zweck, im Mundraum und insbesondere auf den Zähnen einen Flüssigkeitsfilm zu erzeugen, der karieshemmend ist, so daß eventuell verbleibende, auch durch Bürsten nicht entfernte Speisereste auch bei Anwesenheit kariogener Bakterien zu keiner Karies führen können.

Erfindungsgemäß ist es auch vorgesehen, die Lactatdehydrogenase-Wirkung durch Zugabe eines geeigneten Wasserstoffakzeptors, z. B. von Nicotinamidadenin-dinucleotid, gegebenenfalls zu verstärken.

Die Einbringung des Enzyms Lactatdehydrogenase wird zweckmäßigerweise derart durchgeführt, daß sich eine homogene Verteilung innerhalb des Lebensmittels ergibt. Andererseits kann es auch bevorzugt sein, insbesondere dann, wenn das Lebensmittel selbst keine isotrope Zuckerverteilung aufweist, ebenfalls eine ungleichmäßige Verteilung des eingebrachten Enzyms vorzusehen. So wäre es beispielsweise bei Schokolade, die mit ganzen Nüssen durchsetzt ist, nicht erforderlich, den Nußanteil der Schokolade mit Enzym ebenfalls homogen zu durchsetzen.

Die Einbringung von Lactatdehydrogenase erfolgt üblicherweise bei einem pH-Wert, bei dem dieses Enzym stabil ist, sowie bei einer Temperatur, bei der das Enzym nicht denaturiert wird. Ein bevorzugter pH-Bereich liegt zwischen pH 5 bis 7. Besonders bevorzugt ist es, die LDH beim pH-Wert des Stabilitätsoptimums in das Lebensmittel etc. einzubringen. Geeignete Einbringungstemperaturen für LDH liegen zwischen 0 und 50°C, wobei die Temperaturen zwischen 20 bis 50°C als besonders günstig anzusehen sind. Je nach Anwendungszweck kann der Temperaturbereich von 20 bis 40°C besonders bevorzugt sein.

Je nach Art des Mediums kann es wünschenswert sein, das Enzym entweder im Verlauf des Herstellungsverfahrens oder nach Fertigstellung des Lebensmittels oder der pharmazeutischen Zubereitung zuzusetzen. Bei Süßwaren, wie Tafelschokolade, Pralinen etc., ist es gemäß der Erfindung bevorzugt, das Enzym erst zu einem relativ späteren Zeitpunkt zuzufügen, so daß sich keine Aktivitätsverluste im Verlauf der Produktion von Süßwaren ergeben.

Im folgenden werden Versuche beschrieben, die die antikariogene Wirkung bei Zusatz von Lactatdehydrogenase zeigen.

Als kariogenes Substrat wurde ein handelsüblicher, fett- und zuckerhaltiger, wasserarmer Brotaufstrich verwendet. Als Versuchstiere dienten weiße homozygote Ratten vom Typ »Wistar«. Der genannte Brotaufstrich wurde in 50% der Gesamtmenge einem Standard-Trockenfutter (Herilan-RM 20) aus einer vitaminreichen Protein-Fett-Diät, die unter Berücksichtigung aller Stoffwechselbedürfnisse der Ratten entwickelt worden ist, beigemischt.

Die lufttrockene Versuchsdiät wurde proportioniert und so dosiert, daß die Tiere während der Fütterungsperiode von 280 Tagen weiterhin an Gewicht zunehmen konnten. Die Tiere wurden in Kunststoffkäfigen gehalten, die jeweils mit Tränk- und mit Fütterungsautomaten ausgerüstet waren. Dem Trinkwasser wurden in Abständen von 30 Tagen jeweils 50 bis $60 \times 10^3$ Streptococcus mutans-Keime pro Milliliter hinzugesetzt. Als Einstreu diente sterilisiertes Holz-Feingranulat, das in Abständen von 36 Stunden ausgewechselt wurde. Die Raumluft war klimatisiert.

Es wurden Versuchsgruppen gebildet, die aus je 60 Tieren bestanden. Alle Tiere wurden täglich inspiziert und in Abständen von 30 Tagen gewogen, wobei sich keinerlei Nebenwirkungen durch den Gehalt an LDH ermitteln ließen.

Die Feststellungen des kariösen Befundes, insbesondere der kariösen Läsionen, erfolgte mittels Stereomikroskop bei 12facher Vergrößerung.

Folgende Abstufungen wurden notiert:

0 = keine Karies
1 = 1 bis 5 Läsionen
2 = 5 bis 10 Läsionen
3 = mehr als 10 Läsionen.

Geprüft wurden folgende Diätzusammensetzungen:

A = 50% Trockenfutter
    +50% Brotaufstrich
B = 50% Trockenfutter
    +50% Brotaufstrich
    +Lactatdehydrogenase.

Die Lactatdehydrogenasemenge betrug 0,5 mg pro kg Brotaufstrich.

Bei Verfütterung der Diät A traten zahlreiche kariöse Läsionen auf: Von den Versuchstieren waren insgesamt 30 (50%) betroffen. In der nachfolgenden Tabelle sind die Versuchsergebnisse hinsichtlich der Anzahl der Läsionen als auch des jeweiligen Schweregrades zusammengefaßt. Wie die Ergebnisse zeigen, wurde bei Verfütterung der Diät A zum Teil sehr starker Kariesbefall (<10 Läsionen) bei den Versuchstieren festgestellt.

Durch den Zusatz von LDH bei der Diät B wurden sowohl die Zahl als auch der Schweregrad der Läsionen weitgehend reduziert. Dies zeigt deutlich die karieshemmende Wirkung der mit LDH versehenen Diät.

Tabelle

| Versuchsgruppe | Karies-Läsionen (Zahl × Grad) |
|---|---|
| A (Diät A) | 15 × 1; 9 × 2; 6 × 3 |
| B (Diät B) | 6 × 1; 3 × 2 |

Im folgenden werden weitere Beispiele für ein Lebensmittel bzw. eine pharmazeutische Zubereitung angegeben, die einen Zusatz von Lactatdehydrogenase enthalten.

1. Milchschokoladentafel

enthaltend

| | |
|---|---|
| Kakaomasse | 26 g |
| Saccharose | 60 g |
| Milchfett | 3,2 g |
| fettfreie Trockenmasse | 9,5 g |
| Lactatdehydrogenase, die vor dem Erkalten der fertigen Schokolade bei ca. 40 bis 45° C in homogener Verteilung eingerührt worden ist | 5 – 10 mg |

2. Zahnreinigungsdragee

enthaltend Gew.-%

| | |
|---|---|
| Magnesiumcarbonat | 10,0 |
| Siliziumdioxid | 20,0 |
| Dikalziumphosphat | 55,0 |
| Harnstoff-Formaldehyd-Kondensat | 5,80 |
| Aromastoffe | 2,0 |
| Traganth | 1,5 |
| Natriumlaurylsulfoacetat | 2,5 |
| langkettiges Aminfluorid | 2,0 |
| Laktatdehydrogenase | 0,01 |

**Patentansprüche**

1. Karieshemmende, wasserarme, zuckerhaltige, speziell saccharosehaltige Lebensmittel und karieshemmende, wasserarme, zuckerfreie pharmazeutische Zubereitungen, insbesondere Zahn- und Mundpflegemittel, mit einem Gehalt an Lactatdehydrogenase.

2. Karieshemmende Lebensmittel nach Anspruch 1, dadurch gekennzeichnet, daß sie einen Anteil an Saccharose enthalten.

3. Karieshemmende Lebensmittel und pharmazeutische Zubereitungen nach Anspruch 1 oder 2, dadurch gekennzeichnet, daß der Gehalt an Lactatdehydrogenase zumindest ausreichend ist, um die Entstehung kariogener Produkte bzw. die Karies begünstigende Produkte zu unterbinden.

4. Karieshemmende Lebensmittel und pharmazeutische Zubereitungen nach einem oder mehreren der Ansprüche 1 bis 3, dadurch gekennzeichnet, daß sie zur Verstärkung der Lactatdehydrogenase-Wirkung einen geeigneten Wasserstoffakzeptor enthalten.

5. Verfahren zur Herstellung karieshemmender, wasserarmer, zuckerhaltiger Lebensmittel oder karieshemmender, wasserarmer, zuckerfreier pharmazeutischer Zubereitungen nach einem oder mehreren der Ansprüche 1 bis 4, dadurch gekennzeichnet, daß man dem Lebensmittel oder der pharmazeutischen Zubereitung Lactatdehydrogenase bei oder nach Herstellung des Lebensmittels oder der pharmazeutischen Zubereitung zusetzt.

6. Verfahren nach Anspruch 5, dadurch gekennzeichnet, daß man eine derartige Menge an Lactatdehydrogenase verwendet, die die Entstehung von kariogenen Produkten bzw. die Karies mittelbar begünstigende Produkte unterbindet.

7. Verfahren nach einem der Ansprüche 5 bis 6, dadurch gekennzeichnet, daß man die Lactatdehydrogenase in homogener Verteilung einbringt.

8. Verfahren nach einem der Ansprüche 5 bis 7, dadurch gekennzeichnet, daß das Lebensmittel oder die pharmazeutische Zubereitung zum Zeitpunkt des Zusatzes an Enzym im pH-Bereich der optimalen Enzymstabilität gehalten wird.

9. Verfahren nach einem der Ansprüche 5 bis 8, dadurch gekennzeichnet, daß die pharmazeutische Zubereitung oder das Lebensmittel zum Zeitpunkt des Zusatzes an Enzym bei dem pH-Wert 4 bis 7 gehalten wird.

10. Verfahren nach einem der Ansprüche 5 bis 9, dadurch gekennzeichnet, daß man während der Enzymzugabe eine Temperatur von 20 bis 50°C einstellt.

11. Verfahren nach einem der Ansprüche 5 bis 10, dadurch gekennzeichnet, daß man als Lebensmittel wasserarme bzw. -freie Süßwaren verwendet.

12. Verfahren nach Anspruch 5 bis 11, dadurch gekennzeichnet, daß das Lebensmittel ein Tierfutter darstellt.

13. Verfahren nach einem der Ansprüche 5 bis 12, dadurch gekennzeichnet, daß die pharmazeutische Zubereitung Tabletten, Dragees und Mundpflegemittel darstellt.

14. Verfahren nach einem der Ansprüche 5 bis 13, dadurch gekennzeichnet, daß zur Verstärkung der Lactatdehydrogenase-Wirkung ein geeigneter Wasserstoffakzeptor zugegeben wird.

## Claims

1. Caries-inhibiting, low-humidity, sacchariferous, foodstuffs especially containing saccharose, and caries-inhibiting, low humidity pharmaceutical preparations free of sugar, especially dental and mouth washes, having a content of lactate dehydrogenase.

2. Caries-inhibiting foodstuffs according to claim 1, characterized in that they contain a proportion of saccharose.

3. Caries-inhibiting foodstuffs and pharmaceutical preparations according to claims 1 or 2 characterized in that the content of lactate dehydrogenase ist at least adequate to prevent the occurrence of cariogenous products or of products which favor caries.

4. Caries-inhibiting foodstuffs and pharmaceutical preparations according to one or more of claims 1 to 3, characterized in that they contain a suitable hydrogen acceptor to reinforce the lactate dehydrogenase effect.

5. Process for the preparation of caries-inhibiting, lowhumidity sacchariferous foodstuffs or of caries-inhibiting, low humidity pharmaceutical preparations free of sugar according to one or more of claims 1 to 4, characterized in that lactate dehydrogenase is added to the foodstuffs or to the pharmaceutical preparation during or after the manufacture of said foodstuff or pharmaceutical preparation.

6. Process according to claim 5, characterized in that such an amount of lactate dehydrogenase is used as to prevent the incidence of cariogenous products or of products which indirectly favor caries.

7. Process according to one of claims 5 to 6, characterized in that the lactate dehydrogenase is homogenously distributed.

8. Process according to one of claims 5 to 7, characterized in that the foodstuff or the pharmaceutical preparation is kept at the time of addition of the enzyme in the pH range for optimal enzyme stability.

9. Process according to one of claims 5 to 8 above, characterized in that the pharmaceutical preparation or the foodstuffs are kept at a pH value of 4 to 7 at the time of the addition of the enzymes.

10. Process according to one of the claims 5 to 9, characterized in that during the enzyme addition a temperature of 20 to 50°C is maintained.

11. Process according to one of claims 5 to 10, characterized in that low humidity or anhydrous sweet goods are used as the foodstuffs.

12. Process according to claims 5 to 11, characterized in that the foodstuffs concerned are an animal feed.

13. Process according to one of claims 5 to 12 characterized in that the pharmaceutical preparation consists of tablets, pills and mouthwashes.

14. Process according to one of claims 5 to 13 above, characterized in that to reinforce the lactate dehydrogenase effect a suitable hydrogen acceptor is added.

## Revendications

1. Produits alimentaires anti-caries pauvres en eau et renfermant des sucres, en particulier du saccharose, et compositions pharmaceutiques anti-caries pauvres en eau et dépourvues de sucres, plus spécialement produits pour les soins bucco-dentaires, caractérisés en ce qu'ils contiennent une lactate-déshydrogénase.

2. Produits alimentaires anti-caries selon la revendication 1, caractérisés en ce qu'ils contiennet du saccharose.

3. Produits alimentaires anti-caries et compositions pharmaceutiques anti-caries selon l'une des revendications 1 et 2, caractérisés en ce que leur teneur en lactetedéshydrogénase est au moins suffisante pour empêcher la formation de produits générateurs de caries ou de produits favorisant les caries.

4. Produits alimentaires anti-caries et compositions pharmaceutques anti-caries selon l'une quelconque des revendications 1 à 3, caractérisés en ce qu'ils contiennent un accepteur d'hydrogène approprié qui a pour fonction de renforcer l'action de la lactate-déshydrogénase.

5. Procédé de fabrication de produits alimentaires anti-caries pauvres en eau et contenant des sucres ou de compositions pharmaceutiques anti-caries pauvres en eau et dépourvues de sucres selon l'une quelconque des revendications 1 à 4, caractérisé en ce qu'on ajoute au produit alimentaire ou à la composition pharmaceutique une lactate-déshydrogénase au cours de la fabrication du produit alimentaire ou de la composition pharmaceutique ou après cette fabrication.

6. Procédé selon la revendication 5, caractérisé en ce qu'on utilise une quantité de lactate-déshydrogénase qui puisse empêcher la formation de produits cariogènes ou de produits favorisant indirectement les caries.

7. Procédé selon l'une des revendications 5 et 6, caractérisé en ce qu'on introduit la lactate-déshydrogénase de manière à réaliser une répartition uniforme.

8. Procédé selon l'une quelconque des revendications 5 à 7, caractérisé en ce qu'on maintient le produit alimentaire ou la composition pharmaceutique, au moment de l'addition de l'enzyme, dans l'intervalle de pH où la stabilité de l'enzyme est la plus grande.

9. Procédé selon l'une quelconque des revendications 5 à 8, caractérisé en ce qu'on maintient la composition pharmaceutique ou le produit alimentaire, au moment de l'addition de l'enzyme, dans l'intervalle de pH allant de 4 à 7.

10. Procédé selon l'une quelconque des revendications 5 à 9, caractérisé en ce qu'on maintient la température entre 20 et 50°C au cours de l'addition de l'enzyme.

11. Procédé selon l'une quelconque des revendications 5 à 10, caractérisé en ce qu'on utilise, comme produits alimentaires, des sucreries pauvres en eau ou anhydres.

12. Procédé selon l'une quelconque des revendications 5 à 11, caractérisé en ce que le produit alimentaire est une nourriture pour animaux.

13. Procédé selon l'une quelconque des revendications 5 à 12, caractérisé en ce que la composition pharmaceutique est sous la forme de comprimés, de dragées ou de produits pour les soins de la bouche.

14. Procédé selon l'une quelconque des revendications 5 à 13, caractérisé en ce qu'on ajoute un accepteur d'hydrogène approprié pour renforcer l'activité de la lactate-déshydrogénase.